# EUROPEAN PATENT APPLICATION

(11) **EP 2 803 338 A1**
(43) Date of publication of application: **19.11.2014**
(21) Application number: 14166954.9
(22) Date of filing: 05.05.2014
(51) Int. Cl.: A61F 2/848, A61F 2/90

(54) **Medical stent having movement prevention means**

(30) Priority: 15.05.2013 KR 20130055323
(71) Applicant: Standard Sci-Tech Inc., Seoul 130-070 (KR)
(72) Inventor: Jeon, Sang Koo, Namyangju-si, 472-756 Gyeonggi-do (KR); Lee, Sang Hyub, Seongnam-si 463-899 Gyeonggi-do (KR)
(74) Representative: Krauns, Christian

(57) **Abstract**

The present invention provides a medical stent with a stopper (3) which can be inserted into lumens of a human such as the duodenum, the colon, the biliary tract, and the esophagus, and that is useful for lesions due to stricture caused by benign and malignant tumors.

The medical stent with stoppers of the present invention includes a body (1) having a cylindrical shape meshed with an elastic member and having an internal passage, and stoppers (3) forming portions of the body and preventing the body from moving in a lesion, in which the body has a first elastic part (5) at the center thereof, a pair of second elastic parts (7) disposed at respective sides of the first elastic part and extending from the first elastic part, third elastic parts (9) disposed outside of the second elastic parts and extending from the second elastic parts, and fourth elastic parts (11) disposed outside of the third elastic parts, extending from the third elastic parts, and forming ends of the body, the stopper composed of the second elastic parts and the fourth elastic part, and the second elastic parts being larger in diameter than the first elastic part and the third elastic parts.

## Description

### CROSS-REFERENCE TO RELATED APPLICATION

This application claims priority to and the benefit of Korean Patent Application No. 10-2013-0055323 filed in the Korean Intellectual Property Office on May 15, 2013, the entire contents of which are incorporated herein by reference.

### BACKGROUND OF THE INVENTION

### (a) Field of the Invention

The present invention relates to a medical stent with a stopper which can be inserted into lumens of a human such as the duodenum, the colon, the biliary tract, and the esophagus and that is useful for lesions due to stricture caused by benign and malignant tumors.

### (b) Description of the Related Art

In general, when a conduit of an internal organ is narrowed by a lesion on a lumen of a human, a stent operation can be performed to ensure a passage or expand the conduit of the internal organ.

A stent that is used for a stent operation has been disclosed in Korean Patent Laid-Open Publication No. 2010-0090986.

The document discloses a configuration with a locking member on a stent for preventing movement. The locking member extends from the side of the stent and its front end is locked to a lumen so that the stent can keep fixed at the position. The locking member of the stent of the related art is made of a bio-degradable material but has a structure that is physically inserted into a lumen of a human, such that it is difficult to deal with a lesion due to stricture caused by a tumor in the duodenum or the colon.

### [Prior Art Document]

### [Patent Document]

(Patent Document 1) Korean Patent Laid-Open Publication No. 2010-0090986 (published August 18, 2010)

The above information disclosed in this Background section is only for enhancement of understanding of the background of the invention and therefore it may contain information that does not form the prior art that is already known in this country to a person of ordinary skill in the art.

### SUMMARY OF THE INVENTION

The present invention has been made in an effort to provide a medical stent with a stopper that allows the stent to be accurately positioned at the center of a lesion when it is inserted in the duodenum, the colon, the esophagus, or the biliary tract, and that can maximize the effect of medical treatment by preventing the stent from moving from the position.

An exemplary embodiment of the present invention provides a medical stent with stoppers which has a cylindrical shape meshed with an elastic member and having an internal passage, the stoppers forming portions of the body and preventing the body from moving in a lesion.

The body may have a first elastic part at the center, a pair of second elastic parts disposed at respective sides of the first elastic part and extending from the first elastic part, third elastic parts respectively disposed outside of the second elastic parts and extending from the second elastic parts, and fourth elastic parts respectively disposed outside of the third elastic parts, extending from the third elastic parts, and forming the ends of the body.

The stopper may be composed of the second elastic parts and the fourth elastic parts, and the second elastic parts may be larger in diameter than the first elastic part and the third elastic parts.

The fourth elastic parts may be tapered with an end with a larger diameter than the third elastic parts.

The fourth elastic parts may each be provided with a connection wire that is fitted in an elastic member constituting the fourth elastic parts and that reduces the diameter of the fourth elastic parts.

The diameter at the end of the fourth elastic parts may be larger than the diameter of the second elastic parts.

The second elastic parts may be smaller in length than the first elastic part.

The body may be provided with a shielding layer, which is disposed on its inner side and prevents the tissue of a lumen from coming into the body.

The shielding layer may be disposed on the inner sides of the first elastic part, the second elastic parts, and the third elastic parts.

The body may be formed by a wire-shaped elastic member cylindrically wound in a zigzag and making ridges and grooves that are locked to other ridges and grooves by locking structures formed by the elastic member that is repeatedly cylindrically wound in a zigzag.

A radiopaque member may be provided for the first elastic part.

According to the present invention, when the first elastic part that is the center of the body is inserted into the center of a lesion, the first elastic part elastically supports the portion with the lesion, the second elastic members with a diameter larger than that of the first elastic part support the portion around the lesion, and the tapered fourth elastic parts elastically support the lumen of a human with a different elastic force from an elastic force of the first elastic part in the lesion and prevent the stent from separating from the lesion, such that the stent can be better attached to the wall of an intestine and a lesion, and thus the treatment and operation effects can be maximized.

Further, according to the present invention, since a connection line is provided for each of the fourth elastic parts at respective ends of the body, it is possible to easily move the stent using a catheter, such that the first elastic part of the body is positioned at the center of a lesion, when inserting the stent, and accordingly the stent can be precisely inserted.

### BRIEF DESCRIPTION OF THE DRAWINGS

FIG. 1 is a view showing a stent according to an exemplary embodiment of the present invention.
FIG. 2 is a view showing a stent according to another exemplary embodiment of the present invention.

### DETAILED DESCRIPTION OF THE EMBODIMENTS

Hereinafter, exemplary embodiments of the present invention will be described in detail with reference to the accompanying drawings such that those skilled in the art can easily achieve the present invention. As those skilled in the art would realize, the described embodiments may be modified in various different ways, all without departing from the spirit or scope of the present invention. The drawings and description are to be regarded as illustrative in nature and not restrictive. Like reference numerals designate like elements throughout the specification.

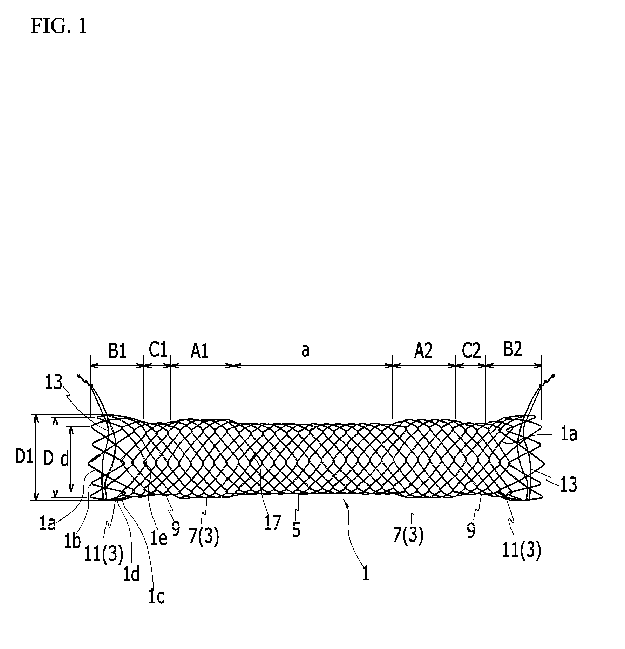

FIG. 1 is a view showing a stent, for illustrating an exemplary embodiment of the present invention.

A stent according to an exemplary embodiment of the present invention includes a cylindrical body 1 and stoppers 3 extending from respective sides of the body 1.

The stoppers 3 include the sections A1, A2 and B1, B2, respectively.

The body 1 according to an exemplary embodiment of the present invention may be a stent that is well-known in the art with a wire-shaped elastic member 1 a cylindrically wound in a zigzag and making ridges 1 b and grooves 1 c. The stent may have a structure with an elastic member 1 a cylindrically repeatedly wound in a zigzag and ridges 1 b and grooves 1 c locked to each other.

The locking structure described above means a structure in which the wire-shaped elastic member makes grooves by bending in a zigzag and make ridges by locking the grooves when it is cylindrically wound and passes the grooves.

The body 1 has a cylindrical shape meshed with the elastic member, and has a long longitudinal internal passage. The body 1 may include a first elastic part 5 (section a), second elastic parts 7 (sections A1 and A2), third elastic parts 9 (sections B1 and B2), and fourth elastic parts 11 (sections C1 and C2).

The stoppers 3, which can maintain the position at the initial position in a lesion without moving when they are inserted in the lesion, and are composed of the second elastic parts 7 (sections A1 and A2) and the fourth elastic parts 11 (sections B1 and B2).

The structural features of the body 1 and the stoppers 3 are described in detail hereafter.

The first elastic part 5 is positioned at the center of the body 1. It is preferable that the first elastic part 5 is disposed at the portion with a lesion.

The second elastic parts 7 are disposed as a pair at respective sides of the first elastic part 5. Obviously, the second elastic parts 7 extend from the first elastic part 5. The second elastic parts 7 have a diameter D that is larger than the diameter d of the first elastic part 5. That is, the second elastic parts 7 expand more than the first elastic part 5.

The third elastic parts 9 extend from a side of the second elastic parts 7. That is, the third elastic parts 9 may have the same diameter d and the same elastic force as those of the first elastic part 5.

The fourth elastic parts 11 form the ends of the body 1, while extending from the third elastic parts 9. The fourth elastic parts 11 are tapered with the ends having a diameter D1 that is larger than the diameter d of the third elastic parts 9.

Further, it is preferable that the fourth elastic parts 11 have the diameter D1 at the ends which is larger than the diameter D of the second elastic parts 7. That is, it is preferable that the elastic force of the fourth elastic parts 11 is larger than the elastic force of the second elastic parts 7 or the third elastic parts 9.

The fourth elastic parts 11 are provided with a connection wire 13. The elastic wire 13 can be put in the ridges formed by the elastic member 1 a formed in a zigzag (which are the portions formed by bending the elastic member at the end of the fourth elastic parts 11). The diameter of the fourth elastic parts 11 can be reduced by pulling the connection wire 13 from the outside. It is preferable to provide the connection wire 13 to each of the fourth elastic parts 11 forming respective ends of the body 1.

Further, it is preferable that the length L2 of the second elastic parts 7 is smaller than the length L1 of the first elastic part 5. The length L2 of one of the second elastic parts 7 is more preferably 40 to 70 % of the length of the first elastic part 5. When the length of the second elastic part 7 is out of the range, the second elastic part may not have sufficient elastic force or may come in contact with a lesion, so the effect of the present invention may be decreased.

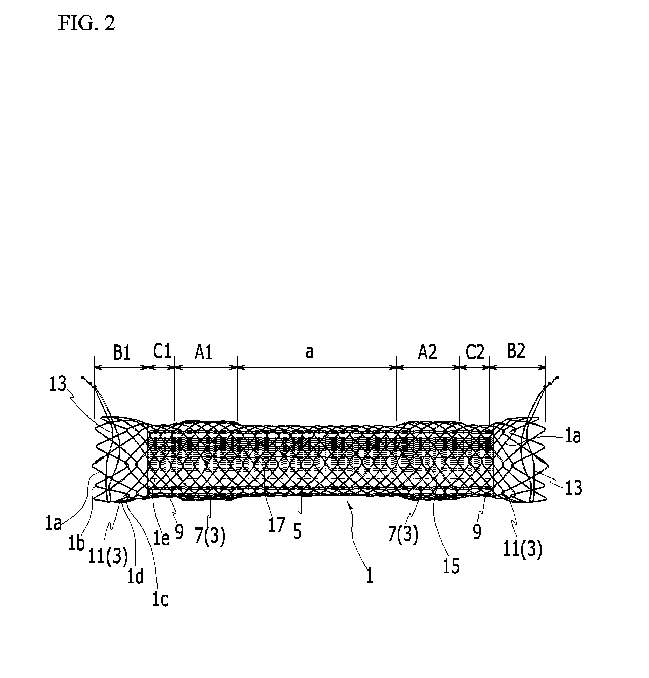

FIG. 2 is a view showing a stent, for illustrating another exemplary embodiment of the present invention. In the description of this exemplary embodiment of the present invention, the description of the previous exemplary embodiment is substituted for the same configurations as those of the previous exemplary embodiment, and only the differences are described.

The current exemplary embodiment of the present invention provides a shielding layer 15 around the inner side of the body 1. The shielding layer 15 is provided to prevent the tissues of a lumen around a lesion of a human from coming into the body 1. It is preferable that the shielding layer 15 is disposed inside the first elastic part 5, the second elastic parts 7, and the third elastic parts 9.

A radiopaque member 17 is provided for the first elastic part 5. The radiopaque member 17 can be seen from the outside so that the first elastic part 5 can be accurately positioned at a lesion when the stent is inserted.

The operation of the stent having the configuration according to an exemplary embodiment of the present invention is as follows.

When it is required to use a stent for a patient, the operator inserts the stent to the position where a lesion in a lumen of a human is present, using a catheter (tool for inserting a stent), and puts it to an appropriate position.

Compared with other parts, the diameter of the lumen of a human with a lesion would be decreased by stricture due to a tumor.

The stent is inserted into the lumen of a human body such that the first elastic part 5 is positioned at the lesion. The second elastic parts 7 apply elastic force to the lesion and other portions away from the lesion.

That is, the first elastic part 5 is disposed at the lesion in a lumen of a human, and is positioned at the portion where the diameter of the lumen is smaller than those of parts other than the lesion. The second elastic parts 7 are disposed in the lumen around the lesion. Accordingly, the elastic force of the first elastic part 5 is applied directly to the lesion having a smaller diameter, and the elastic force of the second elastic parts 7 is applied to the lumen with a larger diameter around the lesion.

Accordingly, the second elastic parts 7 prevent the body 1 from moving. Further, the fourth elastic parts 11 can prevent the body 1 from separating around the lesion, while maintaining close contact with the lumen at a position without the lesion.

When an operator checks the position of a plurality of radiopaque members 17 on the first elastic part 5 from the outside, using X-rays, and the first elastic part 5 is not positioned at the lesion, the operator can adjust the position of the stent by moving the body 1 by pulling the connection line 13.

Further, the shielding layer 15 provided in this exemplary embodiment of the present invention can maximize the operation effect by preventing tissues of human from coming into the stent around a lesion.

As described above, according to an exemplary embodiment, since the stoppers 3 are disposed on the body 1, the stent that has been put at a lesion maintains the posture at the position of the lesion, such that the stent can be attached to the wall of an intestine and a lesion much better, and thus the treatment and operation effects can be maximized.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

### <Description of Symbols>

1. body
1 a. elastic member, 1 b, ridge, 1 c. groove, 1 d. ridge, 1 c, 1 e. groove
3. stopper
5. first elastic part
7. second elastic part
9. third elastic part
11. fourth elastic part
13. connection wire
15. shielding layer
17. radiopaque member

## Claims

1. A medical stent with stoppers, comprising a body having a cylindrical shape meshed with an elastic member and having an internal passage, and stoppers forming portions of the body and preventing the body from moving in a lesion,
wherein the body includes:
a first elastic part at the center thereof;
a pair of second elastic parts disposed at respective sides of the first elastic part and extending from the first elastic part;
third elastic parts respectively disposed outside of the second elastic parts and extending from the second elastic parts, and
fourth elastic parts respectively disposed outside of the third elastic parts, extending from the third elastic parts, and forming the ends of the body,
wherein the stopper is composed of
the second elastic parts and the fourth elastic part, and
the second elastic parts are larger in diameter than the first elastic part and the third elastic parts.

2. The medical stent of claim 1, wherein
the fourth elastic part
is tapered with an end with a larger diameter than the third elastic parts.

3. The medical stent of claim 1 or 2, wherein
the fourth elastic parts are each provided with
a connection wire that is fitted in an elastic member constituting the fourth elastic parts and that reduces the diameter of the fourth elastic parts.

4. The medical stent of any one of claims 1 to 3, wherein the diameter at the end of the fourth elastic parts is larger than the diameter of the second elastic parts.

5. The medical stent of any one of claims 1 to 4, wherein
the second elastic part is
smaller in length than the first elastic part.

6. The medical stent of any one of claims 1 to 5, wherein
the body is provided with
a shielding layer, which is disposed on its inner side and prevents the tissue of a lumen from coming into the body.

7. The medical stent of claim 6, wherein
the shielding layer
is disposed on the inner sides of the first elastic part, the second elastic parts, and the third elastic parts.

8. The medical stent of any one of claims 1 to 7, wherein
the body
is formed by a wire-shaped elastic member cylindrically wound in a zigzag and making ridges and grooves that are locked to other ridges and grooves by locking structures formed by the elastic member that is repeatedly cylindrically wound in a zigzag.

9. The medical stent of any one of claims 1 to 8, wherein a radiopaque member is provided for the first elastic part.

10. The medical stent of any one of claims 1 to 9, wherein the length of the second elastic parts is 40 to 70 % of the length of the first elastic member.
